# FASCICULE DE BREVET EUROPEEN

(11) **EP 2 101 667 B1**
(45) Date de publication et mention de la délivrance du brevet: **11.07.2018**
(21) Numéro de dépôt: 07872032.3
(22) Date de dépôt: 26.12.2007
(51) Int. Cl.: A61B 17/80, F16B 33/02, A61B 17/86

(54) **DISPOSITIF D'OSTEOSYNTHESE COMPRENANT UN SUPPORT AVEC ORIFICE TARAUDE ASSOCIE A UN APPUI, POUR LA RECEPTION D'UNE TIGE D'ANCRAGE**
OSTEOSYNTHESEVORRICHTUNG MIT STÜTZELEMENT MIT INTERNEM BOHRLOCH IN VERBINDUNG MIT EINER TRAGFLÄCHE ZUR AUFNAHME EINES VERANKERUNGSSTABES
OSTEOSYNTHESIS DEVICE COMPRISING A SUPPORT WITH AN INTERNALLY BORED ORIFICE ASSOCIATED WITH A BEARING SURFACE FOR RECEIVING AN ANCHORING ROD

(30) Priorité: 27.12.2006 FR 0611447; 27.12.2006 FR 0611449; 27.12.2006 FR 0611445; 27.12.2006 FR 0611444
(43) Date de publication de la demande: 23.09.2009
(73) Titulaire: D.L.P., 44115 Haute Goulaine (FR)
(72) Inventeur: DEROUET, Guillaume, F-44800 Saint-herblain (FR)
(74) Mandataire: Jacobacci Coralis Harle
(86) Numéro de dépôt international: PCT/FR2007/052624
(87) Numéro de publication internationale: WO 2008/087360

(56) Documents cités:
- EP-A- 1 182 366
- WO-A-00/66045
- US-A1- 2003 083 660
- US-A1- 2005 070 904
- US-A1- 2006 276 793

## Description

La présente invention concerne le domaine des dispositifs destinés aux techniques d'ostéosynthèse ; elle concerne plus particulièrement les dispositifs d'ostéosynthèse du type comprenant un support muni d'au moins un orifice taraudé associé à une surface d'appui, pour la réception d'une tige d'ancrage, laquelle tige comporte une tête prolongée par un corps apte à venir s'ancrer dans le matériau osseux de réception, ladite tête comprenant une partie filetée formant vis et une surface d'appui, le filetage de ladite partie filetée étant complémentaire de celui dudit orifice de support, pour assurer le maintien par vissage de ladite tige sur ledit support, et lesdites surfaces d'appui du support et de la tête de tige étant complémentaires et agencées pour entrer en contact l'une avec l'autre alors que lesdits filetages complémentaires coopèrent entre eux.

La consolidation osseuse de certaines fractures nécessite le montage de matériels ou de dispositifs d'ostéosynthèse sur les différents fragments osseux, pour stabiliser et empêcher une mobilité inter-fragmentaire excessive.

Les praticiens utilisent alors des dispositifs d'ostéosynthèse montés par vissage ou par enfoncement dans les fragments osseux, tels que des vis, broches ou plaques vissées, de sorte à appliquer une compression déterminée et adaptée sur l'os.

Certains de ces dispositifs sont constitués d'une plaque support munie d'un ou de plusieurs orifices, associée à une ou plusieurs tiges insérées chacune dans l'un desdits orifices et destinées à venir s'ancrer dans l'os de réception. La ou les tiges d'ancrage comportent un corps généralement fileté pour son ancrage dans l'os, et une partie supérieure, formant tête, apte à coopérer avec la plaque support.
Dans certains cas, en particulier lorsque l'on cherche à obtenir un verrouillage mono-axial, cette tête de tige comporte un filetage adapté pour coopérer avec un filetage complémentaire ménagé dans l'orifice de réception de la plaque support. En outre, des surfaces d'appui complémentaire ménagées au niveau du support et de la tête de tige, assurent le blocage de la progression de la tige dans le matériau osseux de réception.

De telles structures sont par exemple décrites dans les documents FR-742 618, US-2006/0276793, ou encore US-5 085 660 ;
Mais, dans ce domaine technique, la taille des différentes pièces est minimisée, en particulier pour limiter le traumatisme d'implantation, et les filetages de tige et de support sont très petits.

De ce fait, il est nécessaire de porter une grande attention à l'introduction de la tige dans l'axe de l'orifice du support, pour assurer une coopération correcte entre les filetages, sous peine d'engendrer des contraintes dans les zones de contact, rendant le vissage difficile et risquant d'entraîner des déformations et détériorations des filets. Les matériels actuels ne tolèrent donc pas ou pratiquement pas un défaut de coaxialité entre la tige et l'axe de l'orifice du support, ce qui nuit à la facilité et au confort de pose.

Pour remédier à ces inconvénients, la présente invention propose de tronquer les extrémités saillantes desdits filets de vis (partie filetée de la tige) et de support, ceci de manière beaucoup plus importante que les troncatures fonctionnelles classiques. Plus précisément, tenant compte des valeurs classiques de diamètres qui permettent de définir une vis coopérant avec un orifice taraudé ménagé dans un support (diamètre nominal Dn et diamètre sur flancs Df pour les deux éléments ; diamètre de fond de filet Dff et diamètre extérieur De pour la vis ; diamètre de noyau Dns pour le support), la troncature correspondante est telle que :
- la différence Δtv entre le diamètre extérieur de vis De et le diamètre sur flancs Df est comprise entre 5 et 70 % de la différence Δsv entre le diamètre nominal Dn et le diamètre sur flancs Df, et
- la différence Δts entre le diamètre sur flancs Df et le diamètre de noyau Dns est comprise entre 5 et 70 % de la différence Δss entre le diamètre sur flancs Df et le diamètre de fond de filet Dff.

De préférence, Δtv est comprise entre 15 et 50 % de Δsv, et de manière encore préférée Δtv est de l'ordre de 20 % de Δsv. D'autre part, Δts est de préférence comprise entre 8 et 50 % de Δss, avec une valeur préférentielle de l'ordre de 10 %.

La troncature importante correspondante des extrémités saillantes des filets de vis et de support permet de faciliter l'engrènement desdits filets au moment de l'opération de vissage, ceci tout en améliorant les caractéristiques de robustesse desdits filets.
On obtient un système tolérant qui autorise une petite marge d'erreur en terme de coaxialité à l'origine de l'introduction de la vis dans le support. De plus, la qualité de la tenue mécanique du système vis-support n'est absolument pas handicapée, le transfert de contraintes étant assuré au niveau du diamètre sur flancs.

Les filetages complémentaires du support et de la tête de tige peuvent être simples, doubles, triples, voire plus.

Selon une forme de réalisation particulière, les surfaces d'appui du support et de la tête de tige sont en tronc de sphère. La surface d'appui en tronc de sphère du support est ménagée à la sortie de la partie filetée d'orifice ; la surface d'appui en tronc de sphère de la tête de tige est quant à elle ménagée sous la partie filetée formant vis, dans le prolongement de cette dernière.
Dans ce cadre, l'orifice traversant du support comporte avantageusement (tenant compte du sens d'introduction de la tige) une partie supérieure filetée, surmontant une partie inférieure non filetée définissant la surface d'appui en tronc de sphère.

Selon une autre forme de réalisation particulière, les surfaces d'appui du support et de la tête de tige sont en tronc de cône. La surface d'appui en tronc de cône du support est ménagée au-dessus de l'orifice taraudé ; la surface d'appui en tronc de cône de la tête de tige est quant à elle ménagée au-dessus de la partie filetée formant vis.
Dans ce cadre, l'orifice traversant du support comporte avantageusement (tenant compte du sens d'introduction de la tige) une partie supérieure non filetée, surmontant la partie filetée, laquelle partie supérieure d'orifice comporte la surface d'appui en tronc de cône et assure l'intégration complète de la partie supérieure de la tête de tige, une fois cette dernière convenablement ancrée dans le matériau osseux de réception. Toujours dans ce cadre, le support comporte avantageusement une réservation en forme de surface d'appui sphérique, centrée sur l'axe de l'orifice, aménagée entre la surface d'appui en tronc de cône et la partie filetée dudit orifice. En cas de besoin, le support peut alors recevoir, en remplacement de la tige d'ancrage précitée à verrouillage mono-axial, une vis polyaxiale dont la tête comporte un appui sphérique complémentaire dudit appui sphérique de support.

Selon encore une autre forme de réalisation possible, les surfaces d'appui du support et de la tête de tige sont planes. La surface d'appui plane du support est ménagée à l'entrée de la partie filetée d'orifice ; la surface d'appui plane de la tête de tige est quant à elle ménagée au-dessus de la partie filetée formant vis, ladite surface d'appui plane de la tête de tige définissant la face inférieure d'une embase supérieure de tête.
Dans ce cadre, l'orifice traversant du support comporte avantageusement (tenant compte du sens d'introduction de la tige) une partie supérieure non filetée, surmontant la partie filetée, séparée de cette dernière par la surface d'appui plane précitée, laquelle partie supérieure d'orifice assure l'intégration complète de l'embase supérieure de la tête de tige, une fois cette dernière convenablement ancrée dans le matériau osseux de réception.

Le support consiste avantageusement en une plaque métallique dont l'épaisseur est comprise entre 1 et 5 mm.

L'invention sera encore illustrée, sans être aucunement limitée, par la description suivante, associée aux dessins annexés dans lesquels :
- la figure 1 est une vue schématique, en coupe, d'un dispositif d'ostéosynthèse conforme à la présente invention, comportant un support muni d'un orifice taraudé associé à un appui en tronc de sphère et une tige d'ancrage (ici en forme de vis d'ancrage) ;
- la figure 2 est une vue agrandie d'une partie de la figure 1 détaillant la structure des filetages complémentaires de tige et de support ;
- la figure 3 est une vue schématique, en coupe, d'une variante d'un dispositif d'ostéosynthèse conforme à la présente invention comportant un support muni d'un orifice taraudé associé à un appui en tronc de cône et une tige d'ancrage (ici en forme de vis d'ancrage) ;
- la figure 4 montre l'association du support de la figure 3 avec une vis de type polyaxiale ;
- la figure 5 est une vue schématique, en coupe, d'une autre variante d'un dispositif d'ostéosynthèse conforme à la présente invention, comportant un support avec orifice taraudé associé à un appui plan et une tige d'ancrage (ici en forme de vis d'ancrage).

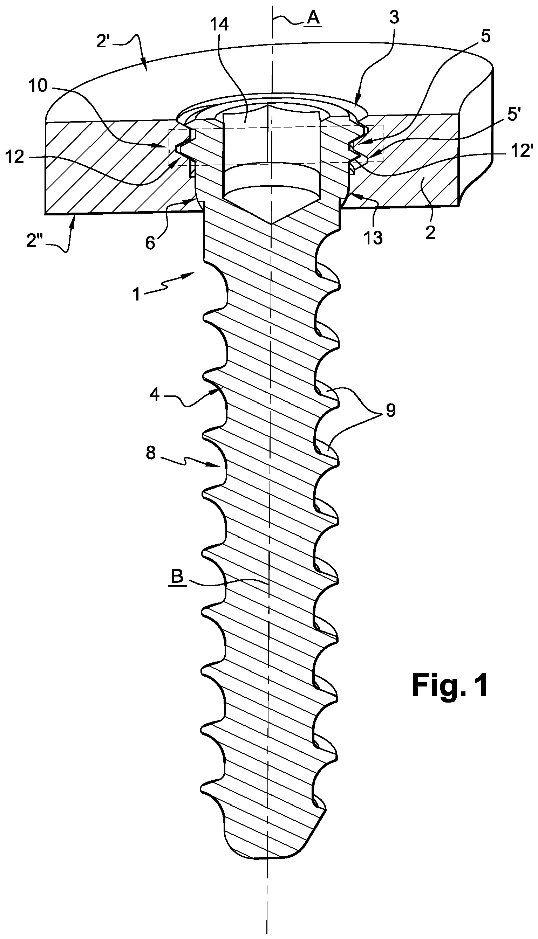

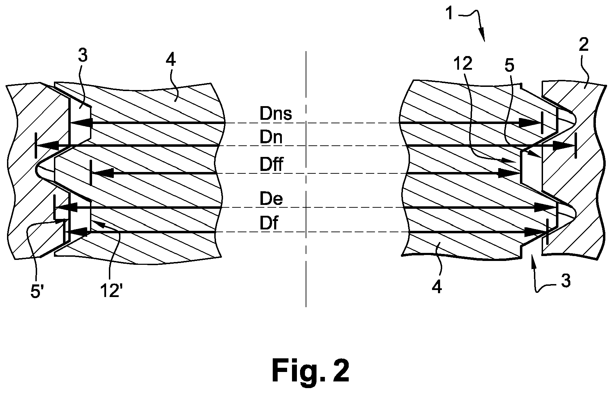

Tel qu'illustré schématiquement sur les figures 1 et 2, le dispositif d'ostéosynthèse 1 comprend un élément support 2 muni d'un orifice traversant 3, pour la réception d'une tige 4 dont l'une des extrémités est adaptée pour venir s'ancrer dans un matériau osseux de réception.

Le support 2 peut prendre toute forme possible. Comme illustré sur la figure 1, il s'agit de préférence d'une plaque métallique de faible épaisseur (par exemple 1 à 5 mm d'épaisseur), présentant une face supérieure 2' et une face inférieure 2", cette dernière étant destinée à venir en contact avec le matériau osseux (non représenté).

L'orifice 3 de réception de la tige 4 est formé d'une partie 5 munie d'un filetage 5', d'axe A, qui débouche au niveau de la face supérieure 2' du support 2, prolongée par une partie non filetée 6, en forme de tronc de sphère, centrée sur ledit axe A, qui débouche au niveau de la face inférieure 2" du support 2. La surface d'appui 6 est ménagée juste à la sortie de la partie taraudée 5.

La tige 4, d'axe B, comporte un corps d'extrémité 8 muni d'un filetage 9, destiné à venir s'ancrer dans le matériau osseux de réception. Son autre extrémité comporte une tête 10 formée d'une partie cylindrique filetée supérieure 12, prolongée en direction du corps d'extrémité 8 par une partie non filetée 13 constituant une surface d'appui en tronc de sphère centrée sur l'axe B de la tige 4. Cette surface d'appui en tronc de sphère 13 est ménagée directement sous la partie filetée 12.

Le filetage 12' de la partie filetée 12 est complémentaire du filetage de support 5', de manière à assurer le maintien par vissage de la tige 4 sur son support 2. Ce filetage 12' peut faire plusieurs tours.

Sur la figure 1, on remarque que la tête de tige 10 comporte une empreinte axiale 14, en forme d'orifice six pans par exemple, destinée aux opérations de vissage (et éventuellement de dévissage) au moyen d'un outil approprié.

Dans une variante de réalisation, le corps d'extrémité 8 de la tige 4 peut avoir une surface externe lisse, dépourvue de filetage.

Comme indiqué précédemment, la présente invention porte sur la structure particulière des filetages 5', 12' complémentaires de support et de tige, et plus particulièrement sur la troncature importante des extrémités saillantes de ces filetages. Les enveloppes de ces filetages 5', 12' sont standard. Ces filetages 5', 12' ne comportent pas de décote entre les flancs par rapport à des filetages classiques ; leur originalité se situe uniquement au niveau de la troncature importante de leurs extrémités saillantes.

Tel qu'illustré sur la figure 2 :
Dn correspond au diamètre nominal du système vis/support, c'est-à-dire au diamètre nominal de la partie cylindrique taraudée 5 de l'orifice 3 et de la partie filetée 12 de la tige 4.
Df correspond au diamètre sur flancs du système vis/support, c'est-à-dire au diamètre sur flancs de la partie cylindrique taraudée 5 de l'orifice 3 et de la partie filetée 12 de la tige 4.
Dff correspond au diamètre de fond de filet de la partie filetée 12 de la tige 4.
Dns correspond au diamètre de noyau de la partie filetée 5 de l'orifice 3 du support 2, et
De est le diamètre extérieur de la partie filetée 12 de la tige 4.

Conformément à l'invention, si :
- Δtv correspond à la différence entre De et Df,
- Δsv est la différence entre Dn et Df,
- Δts est la différence entre Df et Dns, et
- Δss est la différence entre Df et Dff,
alors : Δtv = 5 à 70 % (de préférence 15 à 50 %) de Δsv
et Δts = 5 à 70 % (de préférence 8 à 50 %) de Δss.

Les valeurs préférées de Δtv et Δts sont, respectivement, de l'ordre de 20 % de Δsv et 10 % de Δss.

Les filetages 5' et 12' du dispositif d'ostéosynthèse illustré sur les figures 1 et 2 sont simples. Pour faciliter encore l'insertion de la partie filetée 12 de la tige 4 dans le trou taraudé 5, les filetages en question peuvent être prévus doubles, triples, voire en nombre supérieur à trois.

De manière classique, l'élément support 2 peut comporter une pluralité d'orifices taraudés 3 recevant chacun une tige d'ancrage 4.

Comme illustré sur la figure 1, en fin de vissage, la surface d'appui en tronc de sphère 13 de la tête de vis 10 vient en appui sphérique contre la surface d'appui 6 du support 2. Cet appui sphérique présente des qualités intéressantes de surface de contact et de compacité.

Dans une variante de réalisation possible, les surfaces 6, 13 de contact sphérique entre la tige 4 et le support 2 peuvent être aménagées au-dessus des parties filetées complémentaires 5 et 12. Dans ce cas, la surface d'appui 6 du support 2 est avantageusement prévue à l'entrée de la partie filetée 5.

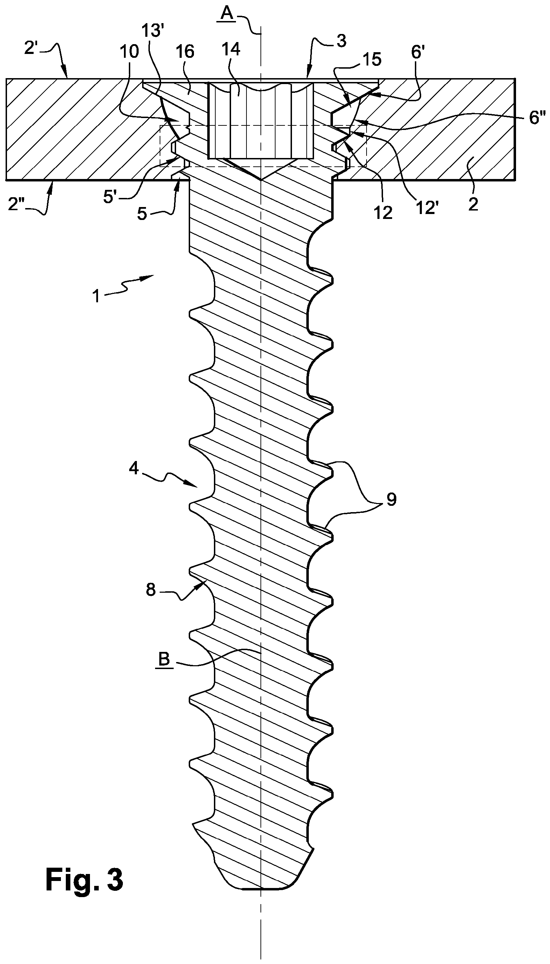

La figure 3 illustre une variante de réalisation d'un dispositif d'ostéosynthèse conforme à l'invention.
Les parties identiques au dispositif illustré sur les figures 1 et 2 conservent les mêmes repères pour simplifier la description.

Ici, l'orifice 3 de réception de la tige 4 est formé d'une partie 5 munie d'un filetage 5', d'axe A, surmontée d'une partie non filetée 15. Cette partie non filetée 15, de diamètre plus grand que la partie filetée 5 débouche au niveau de la face supérieure 2' de la plaque support 2 ; elle se compose d'une partie supérieure 6' (débouchant dans la face supérieure 2' du support 2) ici en forme de surface d'appui tronconique, prolongée, du côté de la partie filetée 5, par une réservation 6" en forme de surface d'appui sphérique.

La surface d'appui tronconique 6' se situe donc au dessus de la partie filetée 5 de l'orifice 3, simplement séparée de l'entrée de cette dernière par la réservation 6", et elle est centrée sur l'axe A de l'orifice 3. La partie filetée 5 débouche au niveau de la face inférieure 2" du support 2.

La surface d'appui sphérique 6" est elle aussi centrée sur l'axe A de l'orifice 3.

La tige 4, d'axe B, comporte un corps d'extrémité 8 muni d'un filetage 9, destiné à venir s'ancrer dans le matériau osseux de réception. Son autre extrémité comporte une tête 10 formée d'une embase supérieure 16 raccordée à une partie filetée 12 sous-jacente, de diamètre plus petit. L'embase 16 de la tête de tige 10 peut avoir une forme générale circulaire ; elle comporte une surface d'appui inférieure 13', ici de forme générale tronconique qui est destinée à venir en contact plan avec la surface d'appui tronconique 6' précitée du support 2. Cette surface d'appui 13' est centrée sur l'axe B de la tige 4.

Là encore, le filetage 12' de la partie filetée 12 est complémentaire du filetage de support 5', de manière à assurer le maintien par vissage de la tige 4 sur son support 2 ; en outre, ces filetages 5' et 12' comportent des extrémités saillantes tronquées tel que décrit ci-dessus en relation avec la figure 2.

Comme illustré sur la figure 3, en fin de vissage, la surface d'appui en tronc de cône 13' de la tête de vis 10 vient en appui conique contre la surface d'appui 6' du support 2 ; de plus, l'embase 16 de cette tête de vis 10 est complètement intégrée dans la partie non filetée 15 du support 2 (dans cette position, la partie supérieure de la tête de vis 10 vient affleurer la face supérieure 2' du support 2).

Cet appui conique présente des qualités de surface de contact et de compacité intéressantes.

Dans une variante de réalisation possible, les surfaces 6', 13' de contact conique entre la tige 4 et le support 2 peuvent être aménagées sous les parties filetées complémentaires 5 et 12. Dans ce cas, la surface d'appui 6' du support 2 est avantageusement prévue à la sortie de la partie filetée 5.

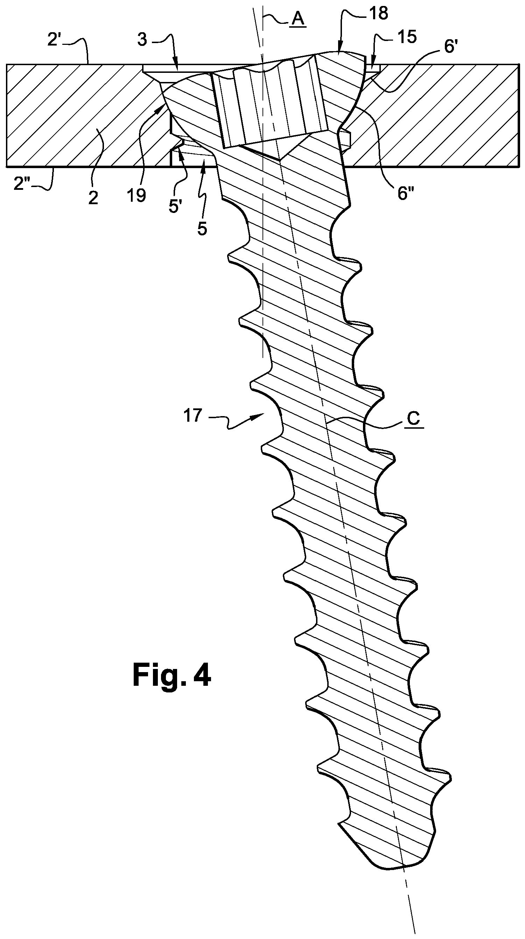

Tel qu'illustré sur la figure 4, si le praticien le souhaite, l'orifice 3 du support 2 peut être équipé, en remplacement de la tige d'ancrage 4 à verrouillage mono-axial, d'une vis polyaxiale 17 dont la tête 18 est munie d'une surface d'appui sphérique 19 complémentaire de l'appui sphérique de support 6".

Cette vis polyaxiale 17 ne comporte pas de filetage susceptible de coopérer avec le filetage d'orifice 5' du support 2 et elle n'est donc pas verrouillée. Elle permet au praticien de mettre en oeuvre des orientations de vis différentes de celles imposées par le système de verrouillage décrit ci-dessus (voir axe C de la vis 17 non confondu avec l'axe d'orifice A sur la figure 4), notamment pour générer des effets de rappel de fragments osseux lors de l'appui par compression de la tête de vis 19 contre le support 2 (par l'intermédiaire de l'appui sphérique 6").

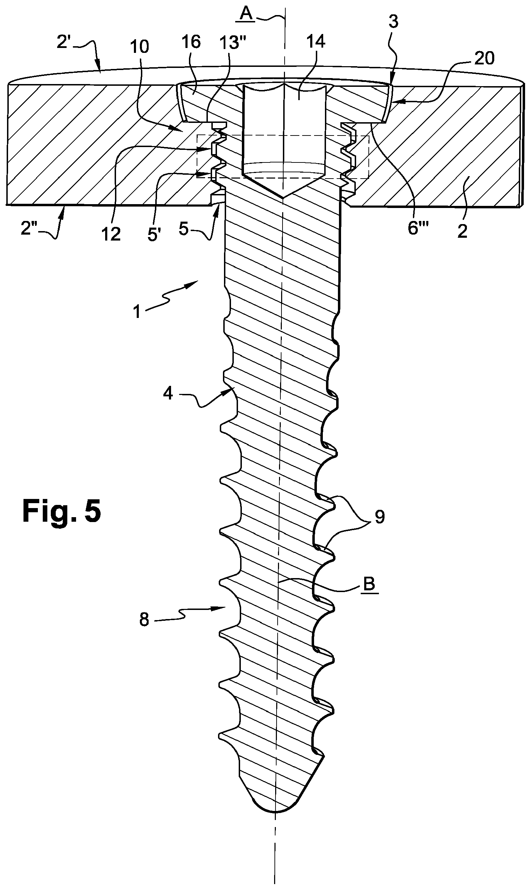

La figure 5 illustre une autre variante de réalisation d'un dispositif d'ostéosynthèse conforme à l'invention.
Ici encore, les parties identiques aux dispositifs illustrés sur les figures 1 à 4 conservent les mêmes repères pour simplifier la description.

Ici, l'orifice 3 de réception de la tige 4 est formé d'une partie 5 munie d'un filetage 5', d'axe A, surmontée d'une partie non filetée 20. Cette partie non filetée 20, de diamètre plus grand que la partie filetée 5 débouche au niveau de la face supérieure 2' de la plaque support 2, elle se raccorde à ladite partie filetée 5 par une surface d'appui plane 6"', de forme générale annulaire, qui s'étend perpendiculairement à l'axe A de l'orifice 3. La surface d'appui plane 6'" est prévue à l'entrée de la partie filetée 5 de l'orifice 3 ; cette partie filetée 5 débouche au niveau de la face inférieure 2" du support 2.

La tige 4 comporte un corps d'extrémité 8 muni d'un filetage 9, destiné à venir s'ancrer dans le matériau osseux de réception. Son autre extrémité comporte une tête 10 formée d'une embase supérieure 16 raccordée à une partie filetée 12 sous-jacente, de diamètre plus petit. L'embase 16 de la tête de tige 10 peut avoir une forme générale circulaire; elle comporte une surface d'appui inférieure 13", qui assure son raccordement avec la partie filetée 12, et qui s'étend perpendiculairement à l'axe B de la tige 4. Cette surface d'appui 13" est destinée à venir en contact plan avec la surface d'appui 6'" précitée du support 2.

Là encore, le filetage 12' de la partie filetée 12 est complémentaire du filetage de support 5', de manière à assurer le maintien par vissage de la tige 4 sur son support 2 ; en outre, ces filetages 5', 12' comportent des extrémités saillantes tronquées de la manière décrite précédemment, notamment en relation avec la figure 2.

Comme illustré sur la figure 5, en fin de vissage, la surface d'appui plane annulaire 13" de la tête de vis 10 vient en appui plan contre la surface d'appui 6'" du support 2 ; de plus, l'embase 16 de cette tête de vis 10 est complètement intégrée dans la partie non filetée 20 du support 2 (dans cette position, la partie supérieure de la tête de vis 10 vient affleurer la face supérieure 2' du support 2).

Dans une variante de réalisation possible, les surfaces 6"', 13" de contact plan entre la tige 4 et le support 2 peuvent être aménagées sous les parties filetées complémentaires 5, 12. Dans ce cas, la surface d'appui 6'" du support 2 est avantageusement prévue à la sortie de la partie filetée 5.

## Revendications

1. Dispositif d'ostéosynthèse comprenant :
- un support (2) muni d'au moins un orifice traversant (3) dont une partie (5) au moins de la hauteur est filetée, laquelle partie filetée (5), d'axe (A), de diamètre nominal Dn, de diamètre sur flancs Df et de diamètre de noyau Dns, est associée, au niveau de son entrée ou de sa sortie, à une surface d'appui (6, 6', 6"'), et
- une tige d'ancrage (4) d'axe longitudinal (B), laquelle tige (4) comporte une tête (10) prolongée par un corps (8) apte à venir s'ancrer dans le matériau osseux de réception, ladite tête (10) comprenant une partie filetée (12) formant vis et une surface d'appui (13, 13', 13"), ladite partie filetée (12) ayant un diamètre nominal Dn, un diamètre sur flancs Df, un diamètre extérieur De et un diamètre de fond de filet Dff, le filetage (12') de ladite partie filetée (12) étant complémentaire de celui (5') dudit orifice de support (3), pour assurer le maintien par vissage de ladite tige (4) sur ledit support (2), et lesdites surfaces d'appui (6, 6', 6"' ; 13, 13', 13") du support (2) et de la tête de tige (10) étant complémentaires et agencées pour entrer en contact l'une avec l'autre alors que lesdits filetages complémentaires (5', 12') à coopèrent entre eux,
**caractérisé en ce que** les extrémités saillantes des filets de vis (12') et des filets de support (5') sont tronquées de telle sorte que :
- la différence Δtv entre le diamètre extérieur de vis De et le diamètre sur flancs Df est comprise entre 5 et 70 % de la différence Δsv entre le diamètre nominal Dn et le diamètre sur flancs Df, et
- la différence Δts entre le diamètre sur flancs Df et le diamètre de noyau Dns est comprise entre 5 et 70 % de la différence Δss entre le diamètre sur flancs Df et le diamètre de fond de filet Dff.

2. Dispositif d'ostéosynthèse selon la revendication 1, **caractérisé en ce que** :
- la différence Δtv entre le diamètre extérieur de vis De et le diamètre sur flancs Df est comprise entre 15 et 50 % de la différence Δsv entre le diamètre nominal Dn et le diamètre sur flancs Df, et
- la différence Δts entre le diamètre sur flancs Df et le diamètre de noyau Dns est comprise entre 8 et 50 % de la différence Δss entre le diamètre sur flancs Df et le diamètre de fond de filet Dff.

3. Dispositif d'ostéosynthèse selon la revendication 2, **caractérisé en ce que** :
- la différence Δtv entre le diamètre extérieur de vis De et le diamètre sur flancs Df est de l'ordre de 20 % de la différence Δsv entre le diamètre nominal Dn et le diamètre sur flancs Df, et
- la différence Δts entre le diamètre sur flancs Df et le diamètre de noyau Dns est de l'ordre de 10 % de la différence Δss entre le diamètre sur flancs Df et le diamètre de fond de filet Dff.

4. Dispositif d'ostéosynthèse selon l'une quelconque des revendications 1 à 3, **caractérisé en ce qu'**il comporte un filetage (5', 12') double.

5. Dispositif d'ostéosynthèse selon l'une quelconque des revendications 1 à 3, **caractérisé en ce qu'**il comporte un filetage (5', 12') triple.

6. Dispositif d'ostéosynthèse selon l'une quelconque des revendications 1 à 3, **caractérisé en ce qu'**il comporte un filetage (5', 12') dont le nombre de filets est supérieur à trois.

7. Dispositif d'ostéosynthèse selon l'une quelconque des revendications 1 à 6, **caractérisé en ce que** les surfaces d'appui (6, 13) du support (2) et de la tête de tige (10) sont en tronc de sphère, **en ce que** ladite surface d'appui (6) en tronc de sphère du support (2) est ménagée à la sortie de la partie filetée d'orifice (5), et **en ce que** ladite surface d'appui (13) en tronc de sphère de la tête de tige (10) est ménagée sous la partie filetée (12) formant vis, dans le prolongement de cette dernière.

8. Dispositif d'ostéosynthèse selon la revendication 7, **caractérisé en ce que** l'orifice traversant (3) du support (2) comporte une partie supérieure filetée (5) surmontant une partie inférieure non filetée (6) définissant la surface d'appui en tronc de sphère.

9. Dispositif d'ostéosynthèse selon l'une quelconque des revendications 1 à 6, caractérisé en que les surfaces d'appui (6', 13') du support (2) et de la tête de tige (10) sont en tronc de cône, en ce que ladite surface d'appui (6') en tronc de cône du support (2) est ménagée au-dessus de l'orifice taraudé (5), et en ce que ladite surface d'appui (13') en tronc de cône de la tête de tige (10) est ménagée au-dessus de la partie filetée formant vis (12).

10. Dispositif d'ostéosynthèse selon la revendication 9, **caractérisé en ce que** l'orifice traversant (3) du support (2) comporte une partie supérieure (15) non filetée, surmontant la partie filetée (5), laquelle partie supérieure d'orifice (15) comporte la surface d'appui en tronc de cône (6') et assure l'intégration complète de la partie supérieure (16) de la tête (10) de tige (4) une fois cette dernière convenablement ancrée dans le matériau osseux de réception.

11. Dispositif d'ostéosynthèse selon l'une quelconque des revendications 9 ou 10, **caractérisé en ce que** le support (2) comporte une réservation (6") entre la surface d'appui en tronc de cône (6') et la partie filetée (5) de l'orifice (3), laquelle réservation (6") est en forme de surface d'appui sphérique centrée sur l'axe (A) de l'orifice (3), ledit orifice (3) étant alors apte à recevoir en remplacement de ladite tige d'ancrage (4) à verrouillage mono-axial, une vis (17) polyaxiale dont la tête (18) comporte un appui sphérique (19) complémentaire dudit appui sphérique de support (6").

12. Dispositif d'ostéosynthèse selon l'une quelconque des revendications 1 à 6, **caractérisé en ce que** les surfaces d'appui (6"', 13") du support (2) et de la tête de tige (10) sont planes, **en ce que** ladite surface d'appui plane (6"') du support (2) est ménagée à l'entrée de la partie filetée d'orifice (5), et **en ce que** ladite surface d'appui plane (13") de la tête de tige (10) est ménagée au-dessus de la partie filetée (12) formant vis, ladite surface d'appui plane (13") de la tête de tige (10) définissant la face inférieure d'une embase supérieure (16) de tête.

13. Dispositif d'ostéosynthèse selon la revendication 12, **caractérisé en ce que** l'orifice traversant (3) du support (2) comporte une partie supérieure (20) non filetée, surmontant la partie filetée (5), séparée de cette dernière par la surface d'appui plane (6"'), laquelle partie supérieure d'orifice (20) assure l'intégration complète de l'embase supérieure (16) de la tête (10) de tige (4) une fois cette dernière convenablement ancrée dans le matériau osseux de réception.

14. Dispositif d'ostéosynthèse selon l'une quelconque des revendications 1 à 13, **caractérisé en ce que** le support (2) consiste en une plaque métallique dont l'épaisseur est comprise entre 1 et 5 mm.

## Patentansprüche

1. Osteosynthesevorrichtung mit:
- einem Träger (2), der mit mindestens einem Durchgangsloch (3) versehen ist, von dessen Höhe wenigstens ein Teil (5) ein Gewinde aufweist, wobei der Gewindeteil (5) mit einer Achse (A) und einem Nenndurchmesser Dn, einem Flankendurchmesser Df und einem Kerndurchmesser Dns im Bereich seines Eingangs oder seines Ausgangs mit einer Auflagefläche (6, 6', 6"') verbunden ist, und
- einer Verankerungsstange (4) mit einer Längsachse (B), wobei die Stange (4) einen Kopf (10) trägt, der durch einen Körper (8) verlängert ist, der geeignet ist, sich in einem Aufnahmeknochenmaterial zu verankern, wobei der Kopf (10) einen eine Schraube bildenden Gewindeteil (12) und eine Auflagefläche (13, 13', 13") aufweist, wobei der Gewindeteil (12) einen Nenndurchmesser Dn, einen Flankendurchmesser Df und einen äußeren Durchmesser De und einen Gewindegrunddurchmesser Dff aufweist, wobei das Gewinde (12') des Gewindeteils (12) zu jenem (5') des Trägerlochs (3) komplementär ist, um das Festhalten der Stange (4) durch Verschrauben auf dem Träger (2) sicherzustellen, und wobei die Auflageflächen (6, 6', 6"'; 13, 13', 13") des Trägers (2) und des Stangenkopfes (10) komplementär und dazu ausgelegt sind, miteinander in Kontakt zu treten, während die komplementären Gewinde (5', 12') miteinander zusammenwirken,
**dadurch gekennzeichnet, daß** die hervorstehenden Enden der Gewindegänge der Schraube (12') und der Gewindegänge des Trägers (5') derart abgeschnitten sind, daß:
- die Differenz Δtv zwischen dem äußeren Schraubendurchmesser De und dem Flankendurchmesser Df zwischen 5 und 70 % der Differenz Δsv zwischen dem Nenndurchmesser Dn und dem Flankendurchmesser Df beträgt, und
- die Differenz Δts zwischen dem Flankendurchmesser Df und dem Kerndurchmesser Dns zwischen 5 und 70 % der Differenz Δss zwischen dem Flankendurchmesser Df und dem Gewindegrunddurchmesser Dff beträgt.

2. Osteosynthesevorrichtung gemäß Anspruch 1, **dadurch gekennzeichnet, daß** :
- die Differenz Δtv zwischen dem äußeren Schraubendurchmesser De und dem Flankendurchmesser Df zwischen 15 und 50 % der Differenz Δsv zwischen dem Nenndurchmesser Dn und dem Flankendurchmesser Df beträgt, und
- die Differenz Δts zwischen dem Flankendurchmesser Df und dem Kemdurchmesser Dns zwischen 8 und 50 % der Differenz Δss zwischen dem Flankendurchmesser Df und dem Gewindegrunddurchmesser Dff beträgt.

3. Osteosynthesevorrichtung gemäß Anspruch 2, **dadurch gekennzeichnet, daß** :
- die Differenz Δtv zwischen dem äußeren Schraubendurchmesser De und dem Flankendurchmesser Df etwa 20 % der Differenz Δsv zwischen dem Nenndurchmesser Dn und dem Flankendurchmesser Df beträgt, und
- die Differenz Δts zwischen dem Flankendurchmesser Df und dem Kerndurchmesser Dns etwa 10 % der Differenz Δss zwischen dem Flankendurchmesser Df und dem Gewindegrunddurchmesser Dff beträgt.

4. Osteosynthesevorrichtung gemäß einem der Ansprüche 1 bis 3, **dadurch gekennzeichnet, daß** sie ein doppeltes Gewinde (5', 12') aufweist.

5. Osteosynthesevorrichtung gemäß einem der Ansprüche 1 bis 3, **dadurch gekennzeichnet, daß** sie ein dreifaches Gewinde (5', 12') aufweist.

6. Osteosynthesevorrichtung gemäß einem der Ansprüche 1 bis 3, **dadurch gekennzeichnet, daß** sie ein Gewinde (5', 12') aufweist, dessen Zahl der Gewindegänge größer als drei ist.

7. Osteosynthesevorrichtung gemäß einem der Ansprüche 1 bis 6, **dadurch gekennzeichnet, daß** die Auflageflächen (6, 13) des Trägers (2) und des Stangenkopfes (10) kugelstumpfartig sind, daß die kugelstumpfartige Auflagefläche (6) des Trägers (2) am Ausgang des Gewindeteils des Lochs (5) angeordnet ist und daß die kugelstumpfartige Auflagefläche (13) des Stangenkopfes (10) unter dem eine Schraube bildenden Gewindeteil (12) in Verlängerung des letzteren gebildet ist.

8. Osteosynthesevorrichtung gemäß Anspruch 7, **dadurch gekennzeichnet, daß** das Durchgangsloch (3) des Trägers (2) einen oberen Gewindeteil (5) aufweist, der einen unteren, nicht mit Gewinde versehenen Teil (6) überragt, der die kugelstumpfartige Auflagefläche definiert.

9. Osteosynthesevorrichtung gemäß einem der Ansprüche 1 bis 6, **dadurch gekennzeichnet, daß** die Auflageflächen (6', 13') des Trägers (2) und des Stangenkopfes (10) kegelstumpfartig sind, daß die kegelstumpfartige Auflagefläche (6') des Trägers (2) oberhalb des Gewindelochs (5) angeordnet ist und daß die kegelstumpfartige Auflagefläche (13') des Stangenkopfes (10) oberhalb des eine Schraube bildenden Gewindeteils (12) gebildet ist.

10. Osteosynthesevorrichtung gemäß Anspruch 9, **dadurch gekennzeichnet, daß** das Durchgangsloch (3) des Trägers (2) einen nicht mit einem Gewinde versehenen oberen Teil (15), der den Gewindeteil (5) überragt, aufweist wobei der obere Teil (15) des Lochs die kegelstumpfartige Auflagefläche (6') trägt und die vollständige Integration des oberen Teils (16) des Kopfes (10) der Stange (4) sicherstellt, sobald letztere im Aufnahmeknochenmaterial sicher verankert ist.

11. Osteosynthesevorrichtung gemäß einem der Ansprüche 9 oder 10, **dadurch gekennzeichnet, daß** der Träger (2) zwischen der kegelstumpfartigen Auflagefläche (6') und dem Gewindeteil (5) des Lochs (3) eine Ausnehmung (6") aufweist, wobei die Ausnehmung (6") die Form einer auf die Achse (A) des Lochs (3) zentrierten kugelförmigen Auflagefläche hat, wobei das Loch (3) dann geeignet ist, als Ersatz für die Verankerungsstange (4) mit einachsiger Verriegelung eine polyaxiale Schraube (17) aufzunehmen, deren Kopf (18) eine komplementäre kugelförmige Auflage (19) der kugelförmigen Trägerauflage (6") aufweist.

12. Osteosynthesevorrichtung gemäß einem der Ansprüche 1 bis 6, **dadurch gekennzeichnet, daß** die Auflageflächen (6"', 13") des Trägers (2) und des Stangenkopfes (10) eben sind, daß die ebene Auflagefläche (6''') des Trägers (2) am Eingang des Gewindeteils des Lochs (5) gebildet ist und daß die ebene Auflagefläche (13") des Stangenkopfes (10) oberhalb des eine Schraube bildenden Gewindeteils (12) gebildet ist, wobei die ebene Auflagefläche (13") des Stangenkopfes (10) die Unterseite eines oberen Kopfsockels (16) definiert.

13. Osteosynthesevorrichtung gemäß Anspruch 12, **dadurch gekennzeichnet, daß** das Durchgangsloch (3) des Trägers (2) einen oberen, nicht mit einem Gewinde versehenen Teil (20) aufweist, der den Gewindeteil (5) überragt und von letzterem durch die ebene Auflagefläche (6'") getrennt ist, wobei der obere Lochteil (20) die vollständige Integration des oberen Sockels (16) des Kopfes (10) der Stange (4) sicherstellt, sobald letztere im Aufnahmeknochenmaterial sicher verankert ist.

14. Osteosynthesevorrichtung gemäß einem der Ansprüche 1 bis 13, **dadurch gekennzeichnet, daß** der Träger (2) aus einer Metallplatte besteht, deren Dicke zwischen 1 und 15 mm beträgt.

## Claims

1. Osteosynthesis device comprising :
- a support (2) provided with at least one through-orifice (3), at least one part (5) of the height of which is threaded, said threaded part (5), of axis (A), nominal diameter Dn, flanks diameter Df and core diameter Dns, being associated, at the entrance or the exit thereof, with a bearing surface (6, 6', 6"'), and
- an anchoring rod (4) of longitudinal axis (B), said rod (4) having a head (10) extended by a body (8) adapted to anchor into the receiving bone material,
wherein said head (10) comprises a screw threaded part (12) and a bearing surface (13, 13', 13"), said threaded part (12) having a nominal diameter Dn, a flanks diameter Df, an outside diameter De and a root diameter Dff, the thread (12') of said threaded part (12) being complementary to that (5') of said support orifice (3), so as to hold said rod (4) on said support (2) by screwing, and said bearing surfaces (6, 6', 6"'; 13, 13', 13") of the support (2) and the rod head (10) being complementary to one another and arranged so as to come into contact with one another when said complementary threads (5', 12') cooperate with each other,
**characterized in that** the protruding ends of said screw threads (12') and support threads (5') are truncated so that:
- the difference Δtv between the outside screw diameter De and the flanks diameter Df is between 5 and 70% of the difference Δsv between the nominal diameter Dn and the flanks diameter Df, and
- the difference Δts between the flanks diameter Df and the core diameter Dns is between 5 and 70% of the difference Δss between the flanks diameter Df and the root diameter Dff.

2. Osteosynthesis device according to claim 1, **characterized in that**:
- the difference Δtv between the outside screw diameter De and the flanks diameter Df is between 15 and 50% of the difference Δsv between the nominal diameter Dn and the flanks diameter Df, and
- the difference Δts between the flanks diameter Df and the core diameter Dns is between 8 and 50% of the difference Δss between the flanks diameter Df and the root diameter Dff.

3. Osteosynthesis device according to claim 2, **characterized in that**:
- the difference Δtv between the outside screw diameter De and the flanks diameter Df is about 20% of the difference Δsv between the nominal diameter Dn and the flanks diameter Df, and
- the difference Δts between the flanks diameter Df and the core diameter Dns is about 10% of the difference Δss between the flank diameter Df and the root diameter Dff.

4. Osteosynthesis device according to any one of claims 1 to 3, **characterized in that** it comprises a double thread (5', 12').

5. Osteosynthesis device according to any one of claims 1 to 3, **characterized in that** it comprises a triple thread (5', 12').

6. Osteosynthesis device according to any one of claims 1 to 3, **characterized in that** it comprises a thread (5', 12') having more than three threads.

7. Osteosynthesis device according to any one of claims 1 to 6, **characterized in that** the bearing surfaces (6, 13) of the support (2) and of the rod head (10) are in the form of a truncated sphere, **in that** said truncated-sphere bearing surface (6) of the support (2) is provided at the exit of the orifice threaded part (5), and **in that** said truncated-sphere bearing surface (13) of the rod head (10) is provided under the screw threaded part (12), in the continuation of the latter.

8. Osteosynthesis device according to claim 7, **characterized in that** the through-orifice (3) of the support (2) comprises a threaded upper part (5) located above a non-threaded lower part (6) defining the truncated-sphere bearing surface.

9. Osteosynthesis device according to any one of claims 1 to 6, **characterized in that** the bearing surfaces (6', 13') of the support (2) and of the rod head (10) are in the form of a truncated cone, **in that** said truncated-cone bearing surface (6') of the support (2) is provided above the threaded orifice (5), and **in that** said truncated-cone bearing surface (13') of the rod head (10) is provided above the screw threaded part (12).

10. Osteosynthesis device according to claim 9, **characterized in that** the through-orifice (3) of the support (2) comprises a non-threaded upper part (15), located above the threaded part (5), said orifice upper part (15) comprising the truncated-cone bearing surface (6') and ensuring the complete integration of the upper part (16) of the head (10) of the rod (4), once the latter correctly anchored in the receiving bone material.

11. Osteosynthesis device according to any one of claims 9 or 10, **characterized in that** the support (2) comprises a re-entrant (6") between the truncated-cone bearing surface (6') and the threaded part (5) of the orifice (3), said re-entrant (6') being in the form of a spherical bearing surface centred on the axis (A) of the orifice (3), said orifice (3) being then adapted to receive, in replacement of said mono-axial locking anchoring rod (4), a poly-axial screw (17) whose head (18) comprises a spherical bearing (19) that is complementary to said support spherical bearing (6").

12. Osteosynthesis device according to any one of claims 1 to 6, **characterized in that** the bearing surfaces (6"', 13") of the support (2) and the head rod (10) are plane surfaces, **in that** said plane bearing surface (6"') of the support (2) is provided at the entrance of the orifice threaded part (5), and **in that** said plane bearing surface (13") of the rod head (10) is provided above the screw threaded part (12), said plane bearing surface (13") of the rod head (10) defining the lower face of a head upper base (16).

13. Osteosynthesis device according to claim 12, **characterized in that** the through-orifice (3) of the support (2) comprises a non-threaded upper part (20), located above the threaded part (5), and separated from the latter by the plane bearing surface (6'"), said orifice upper part (20) ensuring the complete integration of the upper base (16) of the head (10) of the rod (4), once the latter correctly anchored in the receiving bone material.

14. Osteosynthesis device according to any one of claims 1 to 13, **characterized in that** the support (2) consists of a metal plate having a thickness between 1 and 5 mm.
